# EUROPEAN PATENT APPLICATION

(11) **EP 3 273 219 A1**
(43) Date of publication of application: **24.01.2018**
(21) Application number: 17182283.6
(22) Date of filing: 20.07.2017
(51) Int. Cl.: G01N 1/38, G01N 13/00, G01N 17/00, G01N 33/02, G01N 30/00

(54) **APPARATUS AND METHOD FOR EVALUATING SAMPLE SUBSTANCE INTERACTIONS WITH A CONTACT SURFACE**

(30) Priority: 22.07.2016 IT 201600077103
(71) Applicant: TIFQLAB S.r.l., 87036 Rende (CS) (IT); Universita' Della Calabria, 87036 Arcavacata di Rende (IT)
(72) Inventor: ALFANO, Pasquale, 87036 Rende CS (IT); BERARDI, Riccardo, 87036 Rende CS (IT); LAGANÀ, Demetrio, 89129 Reggio Calabria RC (IT); GIAMBELLI, Riccardo, 19013 Deiva Marina SP (IT); BARTOLOMEI, Giovanni, 87036 Rende CS (IT)
(74) Representative: Romano, Giuseppe

(57) **Abstract**

The present invention relates to a preferably mobile apparatus (1) for the treatment of a sample substance (100) for the realization of a direct contact between such sample substance (100) and a contact surface (10) under controlled conditions.

The apparatus (1) comprises a reaction chamber (2) inside thereof the sample substance (100) is contained, the chamber (2) having at least one opening (3) arranged for the realization of a sealed connection between the reaction chamber (2) and the contact surface (10), sensors (5) for the measurement of data related to parameters of the environment inside the chamber (2) and means (6) for adjusting such parameters.

## Description

### Technical field of the invention

The present invention relates to an apparatus for the treatment of a sample substance, in particular a simulant of a food substance, for the realization of a direct contact between such sample substance and a contact surface under controlled conditions.

### Background

In the industrial processes for processing food, this is subjected to mechanical procedures - cooking, cutting, washing, packaging - during which machines and tools of various nature come in direct contact therewith.

All materials and objects intended to come in contact with food are designated with the acronym MOCA, whereas the corresponding field is often designated with the English term *food packaging.*

All objects and machines intended to come in contact with food, or better materials thereof they are made, are compulsory subjected to analytical tests to verify the suitability to the contact with food, the test method thereof is shown in the MD n. 34 of 21 March 1973 "*Hygienic discipline of packagings, recipients, tools, intended to come in contact with food substances or personal use substances*"*.* Instead, as to the materials intended to come in contact with drinking water, the test method is illustrated in the MD n. 174 of 6 April 2004 of the Ministry of Health "*Regulation relating to the materials and objects which can be used in the fixed plants of captation, treatment, adduction and distribution of water intended to the human consumption*"*.*

By means of the analytical tests regulated by the legislations, it is controlled whether or not an interaction between the material and the food intended to the contact therewith takes place, and to which extent.

The checks are due to the fact that the material-food interaction can cause the passage of substances from the material to the food product. This phenomenon, called migration, can be very dangerous, since harmful substances such as the metals could contaminate food and be ingested in quantities dangerous to health.

The risk of yielding material-food substances is evaluated with the so-called global migration tests. Such proofs verify if, and to which extent, substances are yielded from the material to the food destined to the contact, but without identifying specifically the particular yielded component.

A material is considered not suitable to the contact with food if the test provides results of a migration higher than the maximum legal limits.

The migration of a material is influenced by nature and shape of the food product. In particular, to a food product a high extractive capacity is assigned if, by its nature, it combines easily with the materials thereof the packaging is made. For example, a dry food and without fats on the surface has a low extractive capacity, whereas oils and finely ground products in general have a higher extractive capacity.

The migration test according to the currently existing legislations is performed by placing in contact a test piece of the material with the generally liquid samples, which simulate the food intended to the contact.

Generally, the samples include water, acetic acid in aqueous solution, ethanol in aqueous solution and/or olive oil. The liquid selection is dictated by the food nature. The test is performed by reproducing the temperature and time conditions wherein the object intended to the contact with food, or however under the generally considered most rigorous contact conditions, will be used. In particular, repeated tests are provided for tools or containers intended to a use repeated in time.

All tests are performed in specialized laboratories, however there is a problem linked to the check of the real conditions of the machines used in the production plants, which may not correspond to the standard conditions of the tests proposed by the legislations.

In fact, the real environmental and contact conditions can vary from a point to another one of the working line, in a way which cannot be reproduced faithfully in laboratory. For example, in laboratory one does not take into account of the wear of the materials, or the contamination of the surface from the material with other substances possibly present in the working environment which could act as migration catalysts.

Disadvantageously, in most cases, the industrial machines result to be constituted by components which can hardly be disassembled and have too big sizes to be transported in the analysis laboratories, and currently there is not an alternative to the laboratory tests performed on the samples to evaluate the suitability of the materials to come in contact with food.

Therefore, the tests performed on the laboratory samples could be not indicative of the real substance migration from the machines to food during the processing in the industrial production lines.

### Summary of the invention

The technical problem placed and solved by the present invention is then to provide an apparatus for the treatment of a sample substance for the realization of a direct contact between such sample substance and a contact surface under controlled conditions, for the analysis of an interaction by contact between the substance and the surface, allowing to obviate the drawbacks mentioned above with reference to the known art.

Such problem is solved by an apparatus according to claim 1 and by a method according to claim 14.

Preferred features of the the present invention are set forth in the depending claims.

The main advantage of the invention is to provide a more reliable evaluation of the suitability to the contact with food substances of materials intended to the direct contact therewith, in particular way of the machines of the plants for the production and working of food products which cannot be disassembled and transported.

Advantageously, the present invention allows to perform the migration tests on site, directly on the real surface of the machine of the production line intended to the contact with food.

Moreover, the claimed apparatus is equipped with means for the sealed connection with the material to be tested, to guarantee to perform the test under controlled conditions according to the legal parameters.

Still, the apparatus can be provided with moving means, so as it can be transported easily in any point of the production line.

Other advantages, features and use modes of the present invention will result evident from the following detailed description of some embodiments, shown by way of example and not for limitative purposes.

### Brief description of the figures

The figures of the enclosed drawings will be referred to, wherein:
- figure 1 shows a section front view of a first preferred embodiment of the invention;
- figure 2 shows schematically the scheme of the operation principle of a second preferred embodiment of the invention; and
- figures 3A, 3B and 3C show schematically a preferred embodiment of an architecture of a management software of the invention.

The present figures are to be meant exclusively by way of example and not for limitative purposes.

### Detailed description of preferred embodiments

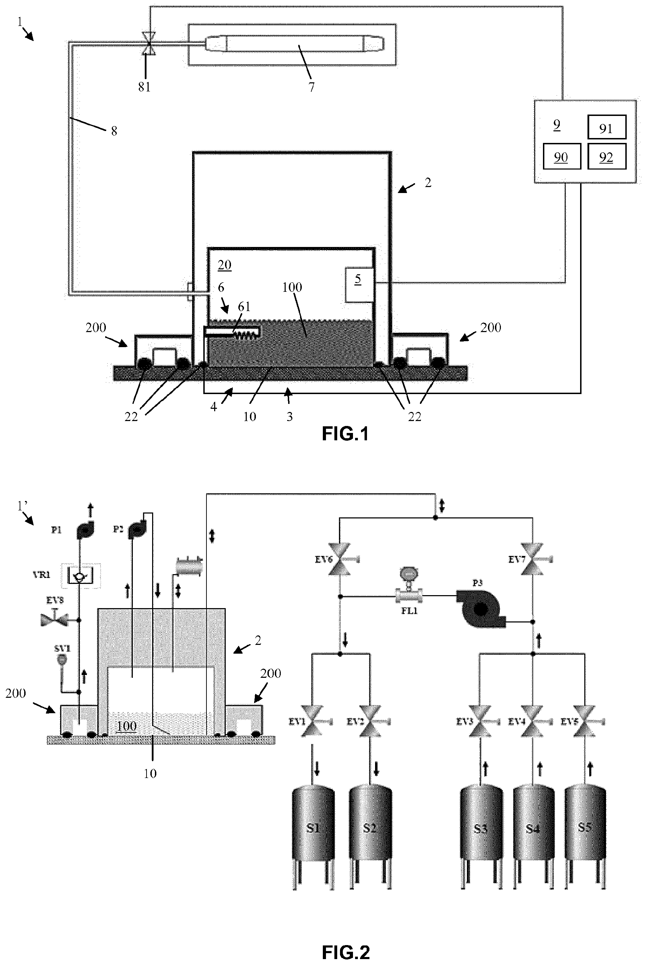

A preferred embodiment of the treatment apparatus of the invention is represented in figure 1, wherein it is designated as a whole with 1.

The apparatus 1 is apt to the treatment of a sample substance 100 for the realization of a direct contact between such sample substance 100 and a contact surface 10, under controlled test conditions.

Such sample substance 100 can be, in particular, a liquid simulating a food substance in liquid form, typically water, acetic acid in aqueous solution, ethanol in aqueous solution, oil. The liquid selection is determined by the nature of the food to be simulated, thereof one wants to evaluate the interaction by direct contact with a determined surface under particular environmental conditions. The test can be performed by reproducing the pressure and temperature environmental conditions and the time of contact with food provided by the legislation, or however by simulating the generally considered most rigorous contact conditions, that is more favourable to the occurrence of the interactions. In particular, repeated tests are provided for tools or containers intended to a use repeated in time.

The conditions, duration and execution modes of the tests depend upon the material of the contact surface 10, upon the food substance and upon the considered contact conditions, according to predetermined test programs which usually follow the dictates of the field legislation.

The apparatus 1 first of all comprises a reaction chamber 2 apt to contain the sample substance 100, that is in particular made at least internally of a material suitable to the contact therewith and so that the interactions by contact with the sample substance under the test conditions are absent or substantially absent.

The chamber 2 has at least one opening 3 comprising a predisposition 4 for the realization of a sealed connection between said reaction chamber 2 and the contact surface 10.

It is important that the connection is sealed to keep the chamber 2 separated with respect to the outer environment and to avoid the contamination of the sample substance 100.

In particular, the predisposition 4 can comprise a nozzle provided with a gasket having a geometry complementary to that of the contact surface therewith the sealed connection is to be implemented during the test.

The chamber 2 delimitates an internal reaction environment 20, wherein the sample substance 100 is kept under controlled environmental conditions, according to the selected test program.

The environmental parameters of the reaction environment 20 are monitored by means of measurement sensors 5, in particular sensors for measuring data related to humidity, pressure and/or temperature.

If the data detected by the sensors 5 are not coherent with the conditions provided by the test program, one can intervene on the adjustment thereof by means of adjustment means 6.

As it can be well seen in figure 1, such adjustment means preferably comprises at least means 61 for heating/cooling the reaction environment 20, for example implemented by means of an electric resistance and a refrigeration circuit.

In addition, such adjustment means can comprise a second pump P2 apt to make the sample substance 100 to bubble in the chamber 2 for simulating particular contact conditions, shown in figure 2.

As shown in figure 1 and more in detail in figure 2, the apparatus 1 can comprise at least a reservoir 7 of the sample substance 100, the reservoir 7 further comprising one or more preferably flexible pipes 8 for feeding the sample substance 100 to the reaction chamber 2.

The feeding of the substance 100 is referred to at least a third pump P3, visible in figure 2.

The pipes 8 comprise means 81 for adjusting the flow - preferably valves for adjusting the flow - of sample substance 100 towards the reaction chamber 2, comprising in particular electrovalves EV (in figure 2 designated with EV1-EV8) and a flowmeter FL1.

According to a preferred embodiment of the apparatus 1' according to the present invention, the number of reservoirs 7 can vary together with the number of sample substances 100 provided according to the different test programs.

The attention is focused on the fact that it is possible feeding to the chamber 2 several sample substances during the same contact test, if a test program requires it, in different quantities and concentrations.

By way of example, the apparatus 1' of figure 2 comprises five reservoirs (designated with S1-S5), each one comprising a sample substance 100. As said before, the flow of sample substance 100 from the reservoirs S1-S5 to the chamber 2 is implemented preferably by using a third pump P3.

The adjustment of the sample substance flow towards the chamber 2 and the adjustment of the parameters of the inner environment 20 can be performed manually, by acting on the adjustment means 6, on the electrovalves EV and on the flowmeter FL1.

Alternatively, according to the preferred embodiment of the invention shown in figure 1, the apparatus 1 can comprise a central control unit 9 implementing such adjustments automatically.

The central unit 9 is configured for acquiring the data detected by the sensors 5, adjusting the operation of the adjustment means 6 as a function of such data and adjusting the operation of the flow adjustment means 81. The unit 9 performs such adjustments according to predetermined test programs, variable based upon the contact surface 10 and the sample substance 100. Preferably, based upon such programs the unit 9 performs an adjustment of initial temperature and end temperature with possibility of varying the temperature according to programmable ramps, but even an adjustment of the test duration with possibility of time programming.

Preferably, the central unit 9 comprises interface means 91 apt to allow a user to enter/select/modify said test programs. Furthermore, in order to simplify such procedure and keep track of the performed tests, the central unit 9 can comprise means 90 for storing the detected data and the test programs.

According to different embodiments, the apparatus 1 can provide even means for washing the feed pipes 8 and/or the reaction chamber 2, for example implemented by means of a circuit fed by a washing fluid, which can be activated automatically by the central unit 9 at the end of a test program and after the picking-up of the sample substance.

According to a not represented additional embodiment of the invention, the reaction chamber 2 can have a second opening for picking up and/or feeding the sample substance 100. The apparatus 1 can comprise automatic means for picking-up the sample substance 100 from the second opening, for example implemented by means of a discharge pipe provided with a third suction pump. Such automatic means is preferably controlled by the central unit 9, which can be further configured for controlling the activation thereof automatically, in particular by actuating the third pump P3, for picking-up the sample substance 100 after the execution of a test program.

The sample substance 100 subjected to the test will be sent to an analysis laboratory for checking the contact interaction during the test, both for evaluating the global migrations (by means of gravimetric analysis) and for checking specific migrations (by analysing the substance with specific instruments based upon the nature of the migrating element to be detected).

In order to allow to monitor the test and to intervene remotely on the central control unit 9, the apparatus 1 can comprise means 92 for connecting the central unit 9 to the Internet network and/or to one or more remote servers.

As shown in both figures 1, 2, the apparatus 1 can comprise advantageously means 22 for moving the reaction chamber 2, to make the transportation thereof easy and quick in the contact positions. For example, the moving means 22 can be wheels placed directly to the chamber 2 or a carriage whereon the chamber is removably installed.

Still, as shown in figures 1 and 2, at least at a side portion of the reaction chamber 2 it is possible to provide the presence of at least a sub-chamber 200. In the embodiments shown in the above-mentioned figures there are two sub-chambers 200, adjacent laterally to the main reaction chamber 2, respectively. Said sub-chambers 200 are configured to keep the reaction chamber 2 pressed on the surface to be analysed, or better integral and adherent thereto. To this purpose, inside the sub-chambers 200 preferably vacuum is made. In particular, at least to one of the above-mentioned sub-chambers 200 pipes can belong for extracting (and re-inserting) the ambient air, for example fed by means of one first pump P1, to implement a circuit preferably comprising an adjustment valve VR1 - preferably a non-return valve - and a safety valve SV1.

By referring to figure 2, the different circuits associated to the main reaction chamber 2 and to one of the above-mentioned sub-chambers 200 adjacent thereto are shown.

In particular, to the main (reaction) chamber 2 the ducts for inletting and extracting the reagents, the duct for balancing the inner pressure, the heating system and the temperature sensors can belong.

The central control unit comprises a logic board which, by inlet/outlet ports, can interface the sensors, the electrovalves, the heating means and all devices which have to be managed and which have to communicate with the hardware control. The software obviously is firmware and it cannot be modified by the end user, which can act according to programmed selections in the user interface.

The system interfaces with the user by means of keyboard, mouse and monitor, like a usual PC.

The system is programmed for all the functions requested by the user, some thereof are detected and enlisted hereinafter:
a) washing of the lines (feed pipes) and/or of the reaction chamber;
b) automatic inletting of the liquids contained in the reservoirs (water, reagents etc.) in the wished quantities and concentrations;
c) adjustment of the initial temperature, end temperature, analysis duration, collection of the obtained sample;
d) possibility of time programming the analysis;
e) possibility of varying the temperature as programmable ramps;
f) remote control of the process, by means of LAN (Internet network) or in case by means GSM/GPRS system.

The software portion allows to control the parameters during the whole migration process. The software has an architecture developed for the mobile apparatus.

The architecture model is that of a SOA system aimed at collecting and consolidating data coming from ubiquitous apparatuses. The system can be based upon a web service, implementation which by now has become standard for the SOA architectures. It guarantees to preserve the fundamental principles of such philosophy, by exposing the functionalities by means of interfaces accessible by heterogeneous systems thanks to the use of common protocols. The operating cycle of a web service is based upon the publication of the functionalities exposed by the service, thereto a request agent can have access according to a known protocol (SOAP or HTML). The proposed implementation is based upon a three-tier architecture, thanks thereto it is possible to preserve the principles of scalability and re-usability of the components, by separating the business logic of the application from the data persistence layer and from the presentation layer. The functionalities of data collection will be exposed by means of web services which can be accessed by sensors through a communication protocol optimized for the transmissions performed by systems subjected to energy saving policies. The collected data are consolidated and stored inside a relational database, scalable too according to the application needs and made redundant with the purpose of increasing safety and effectiveness. The collected data are then processed and shown to the user through a web interface, therefrom the user could perform specific interrogations, as well as display the status of the sensor network. The front end portion allows the use of framework oriented to the RIA (Rich Internet Applications) application. For implementing the web services the selection will be oriented on the REST technologies, by eliminating the heaviness and the overhead caused by the use of the SOAP protocol, used in the homonymous implementation of the web services, by fully exploiting the http potentialities. The relational database, after an accurate study of the data scheme, will have open source matrix too and it will provide support to the layer business through the use of a middleware (JPA) which promotes and increase the separation between the layers.

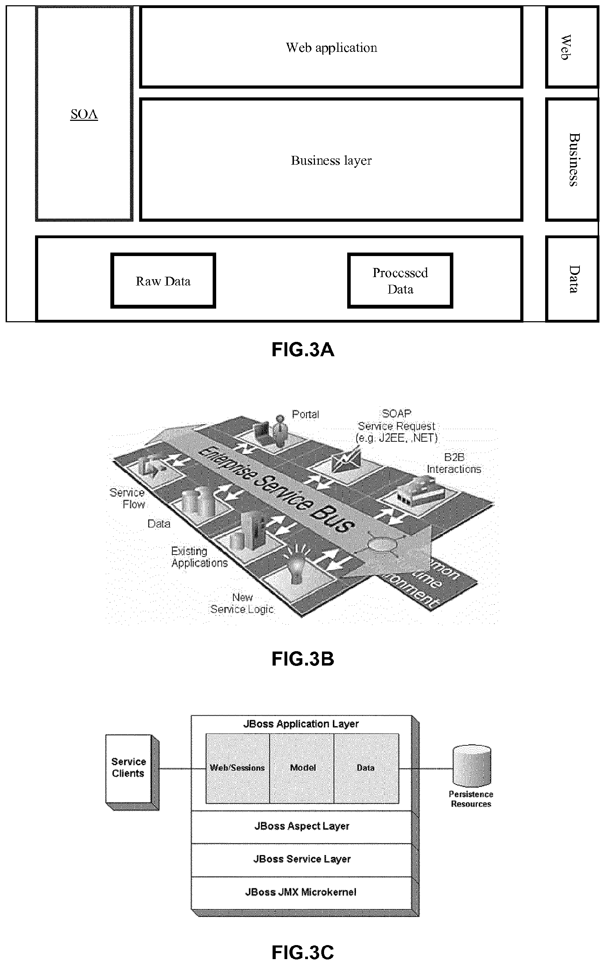

The architecture preferably is of three-tier type, to support a Service Oriented Architecture (SOA), as shown in figures 3A e 3B.

In this way, it is possible to implement REST or XML-based services for reading and writing data on the whole platform.

The Data level is conceptually divided into two macromodules, one assigned to the preservation of the raw data read by the sensor apparatuses and one dedicated to the results of the processing and interrogations, which are usable directly by the upper layers.

An ORM middleware can be used, which allows an automatic mapping between data in relational format and the respective representation to objects. In this way, when it is not necessary to link to the specific functionalities of a particular DBMS, the architecture is wholly independent from the DBMS below.

In particular, the Business level integrates the application logics required to guarantee the correct operation of the whole platform according to the programmed requirements, whereas the Web level makes available a *responsive* user interface, so that it can be used through heterogeneous devices (provided with touch-screen or a pointing system) and with variably sized display.

Among the functionalities of the user level, the following ones are listed:
- checking of the steps for washing the instruments;
- selection of the working parameters;
- checking of the test temperatures and of the start and end times, with the possibility of planning the start time and interrupting a test in any moment;
- checking of the permanence time of the mixture inside the reaction chamber.

### Framework

As far as the SOA sub-system is concerned, it can be implemented by supporting the system with an Enterprise Service Bus, which follows the scheme of the main *best practices* and integration *pattern.*

An example is *switchyard* (ex. http://www.jboss.org/switchyard.html), the new Enterprise Service Bus by JBoss that runs on AS7. Switchyard provides an environment to implement applications focused on Service Oriented Architecture (SOA), an application implemented in such environment, generally, is constituted by one or more components which can provide and/or consume services.

Switchyard, among other things, allows to implement services based upon JAX-RS and JAX-WS, reference specifications as far as the REST and XML-based services are concerned, through visual and easily configurable instruments.

The Data level is implemented by using technologies based upon JPA (Java Persistence API), such as Hibernate. From the point of view of DBMS, technologies such as MySQL or MariaDB can be used, which allow to implement horizontally scalable architectures if required.

Relatively to the Business level, preferably JBoss AS7 (http://www.jboss.org/as7) is used.

JBoss is an open source application server wholly implementing the suite of services offered by JEE (Java Enterprise Edition). Being based upon Java, JBoss is a multiplatform, and it can be installed and used on any system which supports such technology. It allows to implement robust and scalable applications of enterprise level, in the last version its inner architecture has been wholly reviewed, by making it very slim and quick without loosing robustness and reliability. The optimization system exploits the application environment offered by the container JEE of AS7, solution which allows to implement a highly robust and scalable application.

By referring to figure 3C, JBoss architecture can be divided into four levels:
1. Microkernel: it represents the core of the application server, it can use JMX (Java Management Extentions) to perform hot deploy as well as it can have an advance classloading at disposal. Preferably it is structured like a set of MBean (ManagedBeans), so as to be wholly modular and use as less storage as possible.
2. Service Oriented Architecture: it is constituted by a series of services, all self-contained and which can be published on the application server while this is running (hot deploy). Between these services there are transaction services (JTA), messages and safety. In order to improve the performances it is possible to customize this architecture level by removing or adding services.
3. AspectLayer: based upon the programming model oriented to the aspects (AOP). The concept of the interceptors is used, which allow the system to add to the objects the behaviour provided by the services in wholly transparent way through the so-called dependency-injection, typical of framework such as Spring, AspectJ, JBoss AOP.
4. Application Layer: it is the layer whereon there are the applications. Each application can use JBoss potentialities both by invoking directly the container services (SOA) and using the previously described AOP layer.

The WEB level could be implemented through the use of technologies based upon Google Web Toolkit, such as Vaadin (www.vaadin.com). Vaadin, currently arrived at its version 7.6, allows to implement web application with a developing paradigm wholly analogous to that of Java Swing and of similar frameworks. Moreover, starting from its version 7, it integrates natively instruments for implementing wholly responsive applications.

The present invention has been sofar described by referring to preferred embodiments. It is to be meant that other embodiments belonging to the same inventive core may exist, as defined by the protective scope of the here-below reported claims.

## Claims

1. An apparatus (1) for the treatment of a sample substance (100) for the realization of a direct contact between such sample substance (100) and a contact surface (10) under controlled conditions, said apparatus (1) comprising:
- a reaction chamber (2) apt to contain the sample substance (100),
said chamber (2) delimiting an internal reaction environment (20) and having at least one opening (3),
said opening (3) comprising a predisposition (4) for the realization of a sealed connection between said reaction chamber (2) and the contact surface (10);
- sensors (5) for the measurement of data related to parameters of the reaction environment (20); and
- means (6) for adjusting said parameters of the reaction environment (20).

2. The apparatus (1) according to the previous claim, wherein said reaction chamber (2) has a second opening for picking up and/or feeding the sample substance (100).

3. The apparatus (1) according to one of the previous claims, wherein said sensors (5) comprise sensors for measuring temperature, humidity and/or pressure of the reaction environment (20).

4. The apparatus (1) according to one of the previous claims, wherein said adjustment means (6) comprises at least means (61) for heating/cooling the reaction environment (20).

5. The apparatus (1) according to one of the previous claims, comprising at least a reservoir (7) of the sample substance (100), said reservoir (7) comprising one or more pipes (8) for feeding the sample substance (100) to the reaction chamber (2).

6. The apparatus (1) according to the previous claim, wherein said pipes (8) comprise one or more valves (81) for adjusting the flow of said sample substance (100) towards said reaction chamber (2).

7. The apparatus (1) according to the previous claim, comprising a central control unit (9) configured for:
- acquiring the data detected by said sensors (5);
- adjusting the operation of said adjustment means (6) as a function of said data; and
- adjusting the operation of said valves (81),
said adjustments being effected in accordance to predetermined test programs, variable according to the contact surface (10) and the sample substance (100).

8. The apparatus (1) according to the previous claim, wherein said central control unit (9) comprises interface means (91) apt to allow a user to enter/select/modify said test programs.

9. The apparatus (1) according to claim 7 or 8, wherein said central control unit (9) comprises storage means (90) of said data and said test programs.

10. The apparatus (1) according to any of claims 7 to 9, comprising automatic means controlled by said central control unit (9) for picking-up the sample substance (100) from said second opening.

11. The apparatus (1) according to the previous claim, wherein said central control unit (9) is configured to command the activation of said automatic means for picking-up the sample substance (100) after the execution of a test program.

12. The apparatus (1) according to any of claims 7 to 11, comprising means (92) for connecting said central control unit (9) to the Internet network and/or means for connecting to one or more remote servers.

13. The apparatus (1) according to one of the previous claims, comprising moving means (22) of said reaction chamber (2).

14. A method for the preparation of a sample substance (100) for the realization of an interaction by direct contact between the sample substance (100) and a contact surface (10), said method comprising the steps of:
- providing a reaction chamber (2) apt to contain the sample substance (100), said chamber (2) delimiting an internal reaction environment (20) and having at least one opening (3), said opening (3) comprising a predisposition (4) for the realization of a sealed connection between said reaction chamber (2) and the contact surface (10);
- providing sensors (5) for the measurement of data related to parameters of the reaction environment (20);
- providing adjustment means (6) of said parameters of the reaction environment (20);
- implementing a sealed connection between said reaction chamber (2) and the contact surface (10);
- introducing the sample substance (100) inside the reaction chamber (2);
- acquiring data related to the parameters of the reaction environment (20);
- adjusting said parameters by means of said adjustment means (6), as a function of the acquired data and according to a predetermined test program, variable according to the contact surface (10) and the sample substance (100);
- after the execution of a test program, collecting the sample substance (100) from the reaction chamber (2) and
- cleaning the chamber (2) and/or feed pipes 8 from the residues of the sample substance (100) for arranging the apparatus to a new use.
